# EUROPEAN PATENT APPLICATION

(11) **EP 3 984 524 A1**
(43) Date of publication of application: **20.04.2022**
(21) Application number: 20823213.2
(22) Date of filing: 24.03.2020
(51) Int. Cl.: A61K 9/08, A61K 47/02, A61K 31/4164, A61P 33/02, A61P 31/04

(54) **ORNIDAZOLE INJECTION AND S-ORNIDAZOLE INJECTION**

(30) Priority: 13.06.2019 CN 201910509677; 27.09.2019 CN 201910925269
(71) Applicant: Nanjing Y Pharmaceutical Corporation, Nanjing City, Jiangsu 210032 (CN)
(72) Inventor: YU, Qingming, NANJING CITY, Jiangsu 210032 (CN)
(74) Representative: Forresters IP LLP
(86) International application number: PCT/CN2020/080788
(87) International publication number: WO 2020/248648

(57) **Abstract**

The present invention relates to the technical field of injections, especially to an ornidazole injection and an S-ornidazole injection, wherein the ornidazole injection specifically comprises an ornidazole injection and a dilute alkaline solution, and the dilute alkaline solution is an inorganic alkali or an organic alkali. When in use, the ornidazole injection is mixed with the dilute alkaline solution before dripping and the pH value after mixing is 3.5-9.0. The active ingredient can be replaced by S-ornidazole to obtain an S-ornidazole injection. The ornidazole injection and the S-ornidazole injection of the present invention avoid the use of ethanol and propylene glycol excipients that may bring potential risks to patients. The preparation method of the invention is convenient to use, low in preparation cost, and safe in the production process.

## Description

### FIELD OF THE INVENTION

The present invention relates to the technical field of injections, and the specific field is an ornidazole injection and an S-ornidazole injection.

### BACKGROUD OF THE INVENTION

Ornidazole is a third-generation potent drug against anaerobic bacteria infections and against protozoan injections after metronidazole and tinidazole. Its principle of action is to reduce the nitro group in its molecule to an amino group in an oxygen-free environment or through the formation of free radicals, interacting with components in the cell, leading to the death of anaerobic microorganisms. It can treat diseases caused by microbial infections such as anaerobic bacteria, Giardia, Amoeba, Trichomonas and the like. It has the advantages of good drug efficacy, good tissue permeability, and wide distribution in the body. Since its listing, it has been widely used clinically and has good use value.

However, the major limitations of ornidazole and sodium chloride (or glucose) injections as well as S-ornidazole and sodium chloride (or glucose) injections currently used clinically are that they cause pain during injection and even lead to vasculitis. The main reason is the lower pH value of ornidazole and sodium chloride (or glucose) injections as well as S-ornidazole and sodium chloride (or glucose) injections. According to the requirements in the 2000 edition of Chinese Pharmacopoeia, the pH value of ornidazole injections is 3.5-5.5, but in the production process of ornidazole and sodium chloride (or glucose) injections as well as S-ornidazole and sodium chloride (or glucose) injections, they need to be autoclaved and stored, and thus the pH value is greater than 3.5. Related substances will increase, leading to unqualified products. According to the problems and enterprise requirements in the actual implementation process, the standards for ornidazole injections were revised in the 2010 edition of Chinese Pharmacopoeia so as to extend the requirements for the pH value range, which only need to be controlled between 3.0 and 4.0.

The general requirement of Chinese Pharmacopoeia for intravenous preparations is that the pH value is between 4-9. For the particularity of intravenous preparations of ornidazole and sodium chloride (or glucose) injections, the requirements for the pH value range have been extended. For a new standard pH value, in order to ensure product quality, manufacturers choose the standard pH value of infusion preparations to be 3.0 or close to 3.0. For available ornidazole infusion products obtained from some large manufacturers, such as Sichuan Kelun Pharmaceutical Co., Ltd., Xi'an Wanlong Pharmaceutical Co., Ltd. and the like, the determined pH values of the infusions are basically 3.0 or 2.9. For all injection production, the lower limit required by the Chinese Pharmacopoeia is chosen to be the standard pH value. The main reason is that the pH value of an aqueous preparation of ornidazole is in the range of 2.6-4.0, and the lower the pH value is, the more stable the solution will be. However, controlling the pH value of the product to the lower limit of the required pH value range brings considerable difficulty to the control of the production process. The formulating process, autoclave and storage in the circulation field may all cause small changes in pH value. If the pH value is less than 3.0, the product is unqualified.

In order to improve the pain during infusion caused by low pH values, Roche introduced ornidazole in the form of a small volume injection preparation, usually in a dose of 0.5 g to 1 g, and the injection volume is controlled at 3-6 ml. The main principle is that ornidazole has high solubility in organic solvents. When ornidazole is dissolved in organic solvents, autoclave during the production process and long-term storage will not affect the stability of the product, even under higher pH values. Before use, the small volume injection preparation is diluted with physiological saline or glucose injection solution, to be used for an intravenous drip. The main components in the ornidazole small volume injection preparation are propylene glycol, ethanol and a small amount of sodium acetate buffer added to control the pH value between 4.0-4.5. After being diluted with physiological saline or glucose solution before use, the pH value is close to neutral, i.e., 5.0, which improves the above-mentioned adverse reactions of ornidazole injection due to a lower pH value. But its limitation is that the small volume injection preparation introduces the organic solvents, such as ethanol and propylene glycol, which not only increase the production cost, but also introduce propylene glycol as pharmaceutical excipients that are unfavorable to the human body. Such pharmaceutical excipients have certain toxicity. The introduced ethanol can cause disadvantages to ethanol allergic patients. Ethanol and propylene glycol are both organic solvents, especially for ethanol with a low boiling point, which increases potential unsafe risks during the production process

### SUMMARY OF THE INVENTION

The objective of the present invention is to provide an ornidazole injection and an S-ornidazole injection, to solve the problem of pain during the drip and resulted vasculitis due to the lower pH value of ornidazole injections and S-ornidazole injections in the prior art.

In order to achieve the above objective, the present invention provides the following technical solution:
an ornidazole injection, characterized in that it comprises an ornidazole injection and a dilute alkaline solution, and the dilute alkaline solution is an inorganic alkali or an organic alkali.

Wherein, the dilute alkaline solution is a low-concentration trisodium phosphate solution.

Wherein, the concentration of the trisodium phosphate solution is 5-10 mMol/L.

When in use, the ornidazole injection is mixed with the dilute alkaline solution and then dripped, and the pH value after mixing is 3.5-9.0.

Further, the pH value of the ornidazole injection after mixing with the dilute alkaline solution is 5.0-6.0.

Wherein, the ornidazole injection is an ornidazole and sodium chloride injection or an ornidazole and glucose injection. S-ornidazole injections can be obtained by replacing ornidazole in the above injections with S-ornidazole.

According to the present invention, the concentration of the dilute alkaline solution is generally below 1 M, for example 100 mM or 10 mM, preferably 5-10 mM.

According to the present invention, the ornidazole or S-ornidazole injection is used against anaerobic bacteria infections and against protozoan infections.

The present invention provides an ornidazole or S-ornidazole injection, characterized in that it is in the form of a kit, comprising a combination of an ornidazole or S-ornidazole injection and an alkaline solution, and the alkaline solution is an inorganic alkali or an organic alkali. The concentration of the alkaline solution is easily determined and adjusted based on actual needs, such as volume ratio, provided that the pH value of the mixed ornidazole or S-ornidazole injection is within an acceptable range. The inorganic alkali and organic alkali of the present invention are intended to neutralize the ornidazole or S-ornidazole injection, and are not particularly limited, as long as they are physiologically or pharmaceutically acceptable. Wherein, inorganic alkali include, but are not limited to, NaOH, KOH, Na₂CO₃, K₂CO₃, NaHCO₃, KHCO₃, Na₃PO₄, Na₂HPO₄, K₃PO₄, K₂HPO₄ and the like; organic alkali include, but are not limited to, ammonia, sodium acetate, potassium acetate, trisodium citrate, disodium citrate, tripotassium citrate, dipotassium citrate, basic amino acids (such as lysine and arginine), organic amines (such as diethylamine) and the like.

According to the present invention, the volume ratio of the ornidazole or S-ornidazole injection to the alkaline solution is not particularly limited, and mainly depends on the concentration of the alkaline solution. When the concentration of the alkaline solution is higher, its volume is smaller, and when the concentration of the alkaline solution is lower, its volume is larger. The pH value after the two are uniformly mixed is 3.5-9.0, for example, pH 4.0, 4.5, 5.0, 5.5, 6.0, 6.5, 7.0, 7.5, 8.0, 8.5, 9.0, preferably pH 5.0-6.0. In the case of dilute alkaline solution, the volume ratio is 1-2:1 to 1:1-2, preferably about 1:1 (wherein 'about' is ±15%).

Another aspect of the present invention is to provide a method for preparing an ornidazole or S-ornidazole injection, comprising the following steps: preparing an ornidazole or S-ornidazole injection, and an alkaline solution, respectively; before injection, the alkaline solution is added to the ornidazole or S-ornidazole injection with a lower pH value, and the pH value reaches 3.5-9.0, preferably 5.0-6.0 after mixing uniformly. The so-called ornidazole or S-ornidazole injection with 'a lower pH value' means that the pH value is less than 3.5, preferably less than or equal to 3.0, such as 2.7-2.9 and the like.

In the present invention, the dosage form of ornidazole and sodium chloride (or glucose) injections as well as S-ornidazole and sodium chloride (or glucose) injections is changed from one bag per person to two bags per person, wherein one bag is the same as or similar to the original ornidazole and sodium chloride (or glucose) injections as well as S-ornidazole and sodium chloride (or glucose) injections, and another bag of dilute alkaline solution is added. Before injection, add the dilute alkaline solution to the ornidazole and sodium chloride (or glucose) injection as well as the S-ornidazole and sodium chloride (or glucose) injection with a lower pH value, so that the pH value of the mixed injection reaches between 3.5-9.0, preferably between 5.0-6.0. Such dosage form also overcomes the side effects of ornidazole and sodium chloride (or glucose) injections as well as S-ornidazole and sodium chloride (or glucose) injections, which are pain and possible vasculitis due to the lower pH.

The small volume injection preparation from Roche uses ethanol and propylene glycol as solvents, and is diluted with physiological saline or glucose injection solution before the drip. The pH value is close to neutral. Although it overcomes the side effects of pain and resulted vasculitis by low pH, it introduces additional ethanol and propylene glycol, which are unfavorable to the human body.

Dilute alkaline solutions are adopted to adjust pH value of the dosage form of the present invention, avoiding the use of ethanol and propylene glycol excipients that may bring potential risks to patients. The use of dilute alkaline solutions only introduces into the injection low-concentration phosphate that is an essential inorganic salt to the human body and a harmless substance, and the cost thereof is also very low. It is formulated with water for injection. The whole production process does not involve organic solvents and thereby is very safety. During the production of such new dosage forms, there is no need to strictly control the pH value of ornidazole and sodium chloride (or glucose) solution as well as S-ornidazole and sodium chloride (or glucose) solution in bags at 3.0. The pH value can be a bit lower, such as between 2.7-2.9, leading to a better stability and making production process easier to control. The new dosage form is convenient to use, low in preparation cost, and the production process is easy to control and safe.

Compared with the prior art, the beneficial effect achieved by the present invention is that before injecting the ornidazole injection of the present invention, dilute alkaline solution is added to the ornidazole or S-ornidazole injection with a lower pH value, so that the pH value reaches 5.0-6.0; at the same time, it avoids the use of ethanol and propylene glycol excipients that may bring potential risks to patients. The preparation method of the invention is convenient to use, low in preparation cost, and the production process is easy to control and safe.

### DETAILED DESCRIPTION OF THE INVENTION

The technical solutions in the embodiments of the present invention will be clearly and completely described below. Obviously, the described embodiments are only a part of the embodiments of the present invention, rather than all the embodiments. Based on the embodiments of the present invention, all other embodiments obtained by those of ordinary skill in the art without creative work shall fall within the protection scope of the present invention.

### Example 1

Weigh 0.5 g or 1 g of active pharmaceutical ingredient ornidazole, dissolve it in 100 ml of pharmaceutical grade physiological saline, stir to dissolve, adjust the pH value of the solution to 2.75 with hydrochloric acid, filter with a 0.45 micron membrane and fill it into a 250 ml polypropylene infusion bag, autoclave at 121°C for 30 minutes to prepare an ornidazole and sodium chloride solution. Weigh 0.25 g of pharmaceutical grade trisodium phosphate dodecahydrate and 0.81 g of pharmaceutical grade sodium chloride, dissolve them in 90 ml of water for injection (the concentration of trisodium phosphate is about 7.3 mM), filter with a 0.45 micron membrane, fill it into a 150 ml polypropylene infusion bag, and autoclave at 121°C for 30 minutes. Before intravenous drip, add the mixture of trisodium phosphate and sodium chloride in the infusion bag to the ornidazole and sodium chloride injection, shake well, the pH value of the mixed injection is close to 6.0, the concentration of phosphate buffer in the mixture is 3.47 mM.

### Example 2

Weigh 0.5 g or 1 g of active pharmaceutical ingredient ornidazole and 5 g of pharmaceutical grade glucose, dissolve them in 100 ml of water for injection, stir to dissolve, adjust the pH value of the solution to 2.75 with hydrochloric acid, filter with a 0.45 micron membrane and fill into a 250 ml polypropylene infusion bag, autoclave at 115°C for 30 minutes to prepare an ornidazole and glucose solution. Weigh 0.25 g of pharmaceutical grade trisodium phosphate dodecahydrate and 4.5 g of pharmaceutical grade glucose, dissolve them in 90 ml of water for injection (the concentration of trisodium phosphate is about 7.3 mM), fill it into a 150 ml polypropylene infusion bag, and autoclave at 115°C for 30 minutes. Before intravenous drip, add the mixture of trisodium phosphate and glucose in the infusion bag to the ornidazole and glucose injection, shake well, the pH value of the mixed injection is greater than 5.0, and the concentration of phosphate buffer in the mixture is 3.47 mM.

### Example 3

Weigh 0.4 or 0.8 g of active pharmaceutical ingredient S-ornidazole, dissolve it in 100 ml of pharmaceutical grade physiological saline, stir to dissolve, adjust the pH value of the solution to 3.0 with hydrochloric acid, filter with a 0.45 micron membrane, fill it into a 250 ml polypropylene infusion bag, and autoclave at 121°C for 30 minutes to prepare an S-ornidazole and sodium chloride solution. Weigh 0.22 g of pharmaceutical grade trisodium phosphate dodecahydrate and 0.81 g of pharmaceutical grade sodium chloride, dissolve them in 90 ml of water for injection (the concentration of trisodium phosphate is about 6.4 mM), filter with a 0.45 micron membrane, fill it into a 150 ml polypropylene infusion bag, and autoclave at 121°C for 30 minutes. Before intravenous drip, add the mixture of trisodium phosphate and sodium chloride in the infusion bag to S-ornidazole and sodium chloride injection, shake well, the pH value of the mixed injection is close to 6.0, and the concentration of phosphate buffer in the mixture is 3.05 mM.

### Example 4

Weigh 0.4 or 0.8 g of active pharmaceutical ingredient S-ornidazole and 5 g of pharmaceutical grade glucose, dissolve them in 100 ml of water for injection, stir to dissolve, adjust the pH value of the solution to 3.0 with hydrochloric acid, filter with a 0.45 micron membrane, fill it into a 250 ml medical polypropylene infusion bag, and autoclave at 115°C for 30 minutes to prepare an S-ornidazole and glucose solution. Weigh 0.20 g of pharmaceutical grade trisodium phosphate dodecahydrate and 4.5 g of pharmaceutical grade glucose, dissolve them in 90 ml of water for injection (the concentration of trisodium phosphate is about 5.8 mM), fill it into a 150 ml polypropylene infusion bag, and autoclave at 115°C for 30 minutes. Before intravenous drip, add the mixture of trisodium phosphate and glucose in the infusion bag to the S-ornidazole and glucose solution, shake well, the pH value of the mixed injection is greater than 5.0, and the concentration of phosphate buffer in the mixture is 2.8 mM.

### Example 5

The new dosage form of the present invention involves neutralizing relatively acidic ornidazole and sodium chloride (or glucose) solutions as well as S-ornidazole and sodium chloride (or glucose) solutions with dilute alkaline solutions, which is an acid-base neutralization reaction generating neutralization heat, and the heat generated during the mixing process may affect ornidazole and S-ornidazole. Different neutralization heats are produced from the pH value before neutralization to the pH value after neutralization. The two solutions of the new dosage form were equilibrated to 25°C in a constant temperature box, mixed, and the temperature change of the mixed solution in Examples 1 to 4 were measured. Because there is no buffer system in the aqueous solution, the concentration of the added dilute alkaline solution is relatively low. After neutralization, the volume of the mixed solution is also relatively large, and the temperature of the mixed solution only changes slightly. See Table 1, considering the fact that such aqueous solutions can withstand high temperature autoclave, it is believed that the neutralization heat generated during the neutralization process will not cause the destruction of ornidazole and S-ornidazole and the increase of related substances.

**Table 1 Temperature changes of different pH values and pH values after neutralization**

| Example | 1 | 2 | 3 | 4 |
|---|---|---|---|---|
| pH value before neutralization | 2.75 | 2.75 | 3.00 | 3.00 |
| pH value after neutralization | 5 | 6 | 5 | 6 |
| Temperature | 1.9°C | 2.6°C | 1.5°C | 2.3°C |
| increase | | | | |

For further confirmation, the content changes of ornidazole, S-ornidazole and related substances after neutralization were determined. The content determination method was carried out in accordance with the provisions of Appendix V D of the 2010 edition of Chinese Pharmacopoeia with high performance liquid chromatography. The substance to be analyzed is calculated by the peak area according to the external standard method. Analyze the content of the main product and the related substance of thermal decomposition, 2-methyl-5-nitroimidazole. The samples in Examples 2 and 4 with larger temperature changes were taken for measurement, and the samples to be tested were all prepared to a concentration of 50 µg/ml. It can be seen from the results in Table 2 that the main product, related substance and total impurities hardly changed before and after mixing the two solutions of the new dosage form.

**Table 2 Determination of the content of the main product and related substance after mixing the two solutions in Examples 2 and 4**

| Example | Example 2 before mixing | Example 2 after mixing | Example 4 before mixing | Example 4 after mixing |
|---|---|---|---|---|
| Main product (%) | 100.14 | 99.98 | 100.32 | 100.11 |
| 2-methyl-5-nitroimidazole (%) | 0.028 | 0.029 | 0.034 | 0.035 |
| Total impurities (%) | 0.037 | 0.039 | 0.045 | 0.047 |

Clinically, for the use of the new dosage form of the present invention, it is recommended to inject immediately after mixing the two bags of solution. The injection duration generally does not exceed 2 hours. The stability of the mixed injection at different time points after mixing was investigated, as shown in Table 3. Within 24 hours after mixing, the main component and related substance of the drug hardly changed.

**Table 3 Determination of the content of the main product and related substance after two solutions in Example 2 are mixed and placed for different time periods (25°C)**

| Example 2 | after mixing 0h | after mixing 2h | after mixing 8h | after mixing 24 h |
|---|---|---|---|---|
| Main product (%) | 100.17 | 100.32 | 100.27 | 100.12 |
| 2-methyl-5-nitroimidazole (%) | 0.028 | 0.029 | 0.027 | 0.029 |
| Total impurities (%) | 0.038 | 0.041 | 0.039 | 0.040 |

### Example 6

The principle of the new dosage form of the present invention is to neutralize ornidazole and sodium chloride (or glucose) solutions as well as S-ornidazole and sodium chloride (or glucose) solutions with lower pH values with dilute alkaline solutions. The concentration of the dilute alkaline solution is very low, only at a level of a few mM. The dilute alkaline solution is safer at room temperature. If it touches the human body, it can be washed with tap water. However, for the preparation of injections, dilute alkaline solutions need to be autoclaved at high temperatures, and medical packaging materials may have problems with their tolerance to alkaline solutions at high temperatures, which may cause the packaging materials to degranulate and fail to meet the requirements of injections.

The medical polypropylene bag is used as the packaging material of dilute alkaline solution, and preliminary verification has been carried out. Take 5 ml of samples, use a particle size instrument to test the number of particle sizes of 10 and 25 microns during the sample preparation process and under different storage conditions. The samples to be tested are the samples after autoclave. After autoclave, they are placed at different temperatures, such as 60°C and 25°C for different time periods, and the changes in the number of particles are shown in Table 4. The results show that when the dilute alkaline solution in the new dosage form of the present invention is packed in the polypropylene medical infusion bag packaging material under the conditions of chemical production, the number of particles meets the requirements of injections.

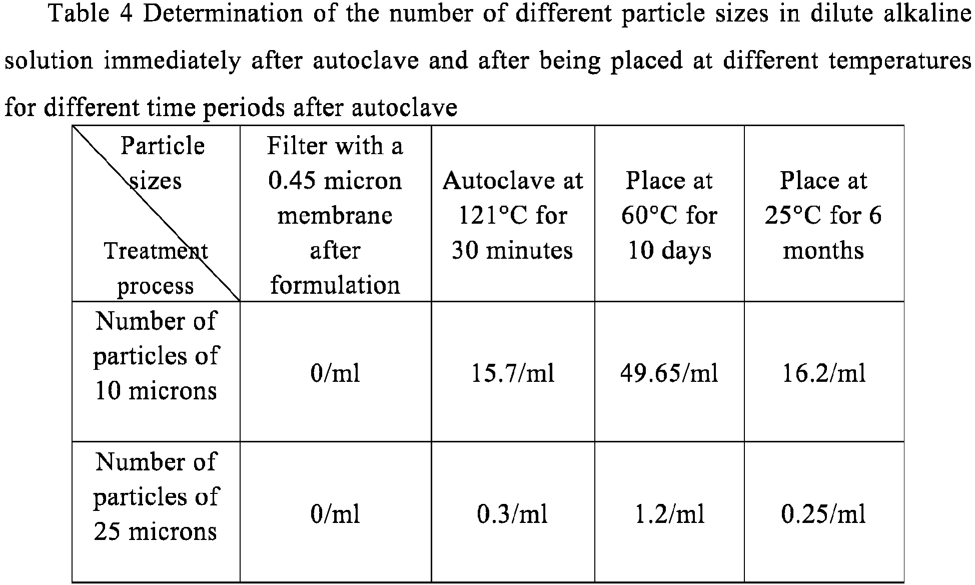

Although the embodiments of the present invention have been shown and described, those of ordinary skill in the art can understand that various changes, modifications, substitutions, and variations can be made to these embodiments without departing from the principle and spirit of the present invention. The scope of the present invention is defined by the appended claims and their equivalents.

## Claims

1. An ornidazole injection, **characterized in that** it comprises an ornidazole injection and a dilute alkaline solution, and the dilute alkaline solution is an inorganic alkali or an organic alkali.

2. The ornidazole injection according to claim 1, **characterized in that** the dilute alkaline solution is a low-concentration trisodium phosphate solution.

3. The ornidazole injection according to claim 2, **characterized in that** the concentration of the trisodium phosphate solution is 5-10 mMol/L.

4. The ornidazole injection according to claim 3, **characterized in that** when in use, the ornidazole injection is mixed with the dilute alkaline solution and then dripped, and the pH value after mixing is 3.5-9.0.

5. The ornidazole injection according to claim 4, **characterized in that** the pH value after mixing the ornidazole injection with the dilute alkaline solution is 5.0-6.0.

6. The ornidazole injection according to any one of claims 1 to 5, **characterized in that** the ornidazole injection is an ornidazole and sodium chloride injection or an ornidazole and glucose injection.

7. An S-ornidazole injection, **characterized in that** ornidazole in claims 1-6 is replaced by S-ornidazole.

8. The ornidazole injection according to any one of claims 1-6, **characterized in that** the ornidazole injection and the dilute alkaline solution is in a volume ratio of 1-2:1 to 1:1-2, preferably about 1:1.

9. The ornidazole injection according to any one of claims 1-6 and 8 or the S-ornidazole injection according to claim 7, for use against anaerobic bacteria infections and against protozoan infections.

10. An ornidazole or S-ornidazole injection, **characterized in that** it comprises a combination of an ornidazole or S-ornidazole injection and an alkaline solution, and the alkaline solution is an inorganic alkali or an organic alkali.

11. A method for preparing an ornidazole or S-ornidazole injection, comprising the following steps:
preparing an ornidazole or S-ornidazole injection, and an alkaline solution, respectively;
before injection, the alkaline solution is added to the ornidazole or S-ornidazole injection with a lower pH value, and the pH value reaches 3.5-9.0, preferably 5.0-6.0 after mixing uniformly.
